# EUROPEAN PATENT SPECIFICATION

(11) **EP 0 344 983 B1**
(45) Date of publication and mention of the grant of the patent: **15.09.1993**
(21) Application number: 89305342.1
(22) Date of filing: 26.05.1989
(51) Int. Cl.: C07D 333/38

(54) **Process for preparing 2- and 4-alkoxycarbonyl thiolan-3-ones**
Verfahren zur Herstellung von 2-und 4-alkoxycarbonyl Thiolan-3-onen
Procédé de préparaton des thiolan-3-ones 2- et 4-alcoxycarbonyliques

(30) Priority: 27.05.1988 GB 8812718
(43) Date of publication of application: 06.12.1989
(73) Proprietor: JOHN WYETH & BROTHER LIMITED, Maidenhead Berkshire, SL6 0PH (GB)
(72) Inventor: Shepherd, Robin Gerald, Windsor Berkshire, SL4 1SH (GB)
(74) Representative: Wileman, David Francis, Dr.

(56) References cited:
- EP-A- 0 032 748
- JOURNAL OF THE AMERICAN CHEMICAL SOCIETY vol. 68, November 1946, pages 2229-2235, Columbus, Ohio, USA; R B Woodward et al.: "Tetrahydrothiophene ("Thiophane") Derivatives"
- MONATSHEFTE FUER CHEMIE vol. 104, pages 1520-1525, Springer-Verlag 1973; O Hromatka et al.: "über den Mechanismus der Dieckmann-Reaktion von 3-(Methoxycarbonylmethylthio)-prpionsäur emethylester"
- THE JOURNAL OF ORGANIC CHEMISTRY vol. 45, no. 4, 15 February 1980, pages 617-620, Columbus, Ohio, USA; P A Rossy et al.: "Aromatization of Dihydrothiopes. Thiophenesaccharin : A sweet Surprise"
- ARKIV FOER KEMI vol. 11, no. 59, 5 June 1957, pages 527-533; A Braendstroem : "On the reactivity of ions and ion-pairs in the Dieckmann condensation"
- TETRAHEDRON LETTERS vol. 22, no. 14, 1981, pages 1353-1354; Y Yamada et al.: "A direction controlled Dieckmann Type cyclization of half-thiol diesters"

## Description

This invention relates to a novel process, more particularly to a novel process for the preparation of 2- and 4-alkoxycarbonylthiolan-3-ones which are useful chemical intermediates.

Certain 2 and 4-alkoxycarbonylthiolan-3-ones of the type: 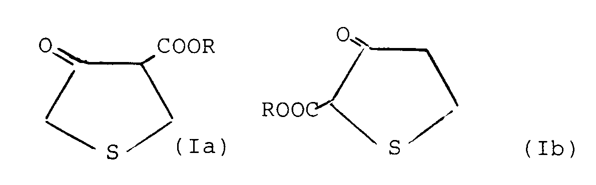
wherein COOR represent an ester group such as methoxycarbonyl or ethoxycarbonyl are known or are analogous to known compounds described in the chemical literature. These compounds in general have utility as chemical intermediates.

For example, 4-ethoxy- and 4-methoxy-carbonylthiolan-3-ones have been used as intermediates in drug syntheses and in the preparation of sweeteners - see for example R K Razdan et al, J. Med. Chem.,19, 549 (1976);P A Rossy et al, J. Org. Chem., 45, 617 (1980) and United States Patent No. 4143050 published 6 March 1979.

Similarly 2-methoxycarbonylthiolan-3-one and other esters are described in US Patent No 4143050 as useful in the preparation of starting materials for the manufacture of drugs and sweeteners.

The preparation of 4-methoxycarbonylthiolan-3-one via a Dieckmann cyclisation of 3-methoxycarbonylmethylthiopropanoic acid methyl ester (A) is described by P A Rossy et al, in J. Org. Chem., 45, 617 (1980) at page 619 and involves the use of sodium methoxide in methanol to give the product in a yield from 50-55%. A similar reaction giving 4-methoxycarbonylthiolan-3-one in 67.5% yield is described by Hromatka et al in Monatsh, Chem., 104, 1520-1525 1973. R B Woodward and R H Eastmann in J. Amer. Chem. Soc, 2229 (1946) also describe the Dieckmann cyclisation of the same starting material but using sodium methoxide in dry ether at room temperature to give 2-methoxycarbonylthiolan-3-one. However, at 80°-120°C using NaOMe in dry toluene Woodward and Eastmann found the main product was 4-methoxycarbonylthiolan-3-one prepared in about 49% yield (ca.30% isolated).

F Duus, in Tetrahedron Vol 37, No 15 pp 2633 to 2640 (1981) describes the preparation of 4-ethoxycarbonylthiolan-3-one in 35% yield via a Dieckmann condensation of 3-ethoxycarbonylmethylthiopropanoic acid ethyl ester using a suspension of sodium ethanolate in boiling toluene. When ethanol was used as solvent and the reaction temperature was lowered to 0°C the product obtained was 2-ethoxycarbonylthiolan-3-one in 23-43% yield.

It has now been found that by using lithium alkoxides in the Dieckmann cyclisation of 3-alkoxycarbonylmethylthiopropanoic acid esters then excellent yields of either the 2- or 4-alkoxycarbonylthiolan-3-ones or mixtures thereof can be obtained by choice of appropriate reaction times and temperatures.

The use of lithium alkoxide gives advantages over sodium alkoxide which include higher yields and purer products. In addition the reaction may be carried out substantially free from unpleasant odours which occur when sodium alkoxide is used.

Further if desired the 4-alkoxycarbonylthiolan-3-ones can be conveniently prepared substantially free from contamination by the corresponding 2-alkoxycarbonyl isomer and vice versa.

Accordingly in one aspect this invention provides a process for preparing a compound of formula Ia or Ib 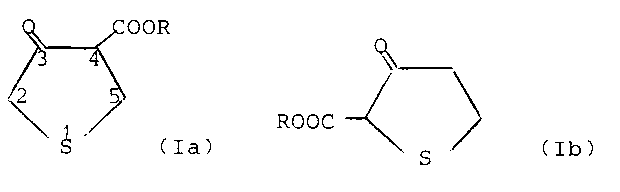
or a lithium salt or mixtures thereof, which comprises cyclising a compound of formula 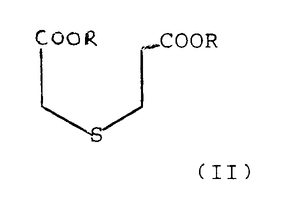
wherein COOR represents the same or different ester functions, in the presence of a lithium alkoxide, eg.alkoxides of 1 to 6 carbon atoms such as lithium methoxide and if desired acidifying to give the compound of formula Ia or Ib or mixtures thereof.

In the aforementioned process the reaction may be effected quickly in a time which is sufficiently short to produce the lithium salt of the compound of formula Ib substantially in excess of or preferably substantially free from, the corresponding lithium salt of the compound of formula Ia.

Low reaction temperatures eg. less than about 50°C, such as room temperature or below, also assist in obtaining the lithium salt of the compound of formula Ib by slowing its conversion to formula Ia.

However where it is desired to obtain the lithium salt of the compound of formula Ia substantially in excess of formula Ib then higher reaction temperatures, eg. above about 50°C or more, and/or longer reaction times eg. about 3 hours or more, depending on the reaction temperature, may be used.

Mixtures may be obtained at intermediate times and temperatures from which individual 2- or 4- esters of formula Ia and Ib may be isolated by standard techniques. For the preparation of mixtures, the process of this invention employing lithium alkoxide is superior to use of sodium alkoxide in view of the high yields of purer combined products and the absence of obnoxious odours.

In a preferred aspect this invention provides a process for preparing a compound of formula Ia or a lithium salt thereof, which comprises cyclising a compound of formula II as defined above in the presence of a lithium alkoxide, eg. alkoxides of 1 to 6 carbon atoms such as lithium methoxide and for a time sufficient to produce the product substantially free from the 2-isomer and if desired acidifying to give the compound of formula Ia.

In the formulae Ia, Ib and II COOR may represent any ester function that is stable under the reaction conditions. Preferred values for R are alkyl eg. alkyls of 1 to 6 carbons (such as methyl, ethyl, propyl and butyl) and aralkyl such as aralkyl of 7 to 12 carbon atoms, eg. benzyl and phenethyl.

Preferably both COOR groups are the same eg. methyl or ethyl. Most preferably the lithium alkoxide has the formula LiOR where R is the same group as R in the compound of formula II.

The use of lithium alkoxides is also particularly advantageous since extended reaction times may be employed without deterioration of reaction product of formula Ia. This is especially important if it is desired to carry out the reaction on a large scale or in the plant where transfer and handling of bulk materials makes it difficult to achieve short reaction times.

The reaction is conveniently carried out in a solvent comprising an alcohol, eg. ROH such as ethanol. A hydrocarbon and/or linear or cyclic ether may also be present, eg. toluene, tetrahydrofuran or dioxane.

Sodium alkoxides under hot conditions degrade 2- and 4-akloxycarbonylthiolan-3-ones with the result that short reaction times are necessary if an optimum yield is desired.

This invention also provides the lithium salts of compounds of formulae Ia and Ib as defined hereinabove. A preferred compound of the invention is the lithium salt of 4-methoxycarbonylthiolan-3-one. Acidification of the lithium salts provide compounds of formulae Ia and Ib. Accordingly this invention provides a process for preparing a compound of formula Ia or Ib which comprises acidifying the corresponding lithium salt.

The following route is described in J. Org. Chem. 45, 617 (1980) for preparing a sweetening agent, thiophene saccharin(B), sodium salt, using 4-methoxycarbonylthiolan-3-one:

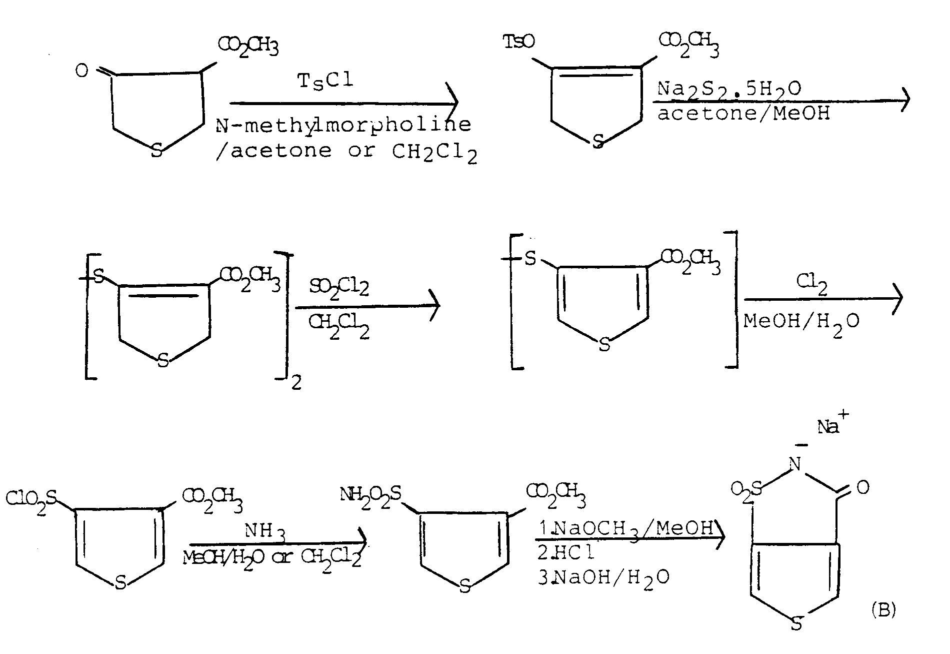

Similarly other esters of formula Ia may be used. Accordingly in a further aspect this invention provides thiophenesaccharin when prepared from a compound of formula Ia made according to a process of this invention.

The following non-limiting Examples illustrate the invention:

### EXAMPLE 1

### 4-Methoxycarbonylthiolan-3-one

a) Lithium (8.46g, 1.245M) in toluene (11) was treated with methanol (315ml). After all the lithium had dissolved, 3-methoxycarbonylmethylthiopropionic acid methyl ester (216g 1.12M) was added over 0.5 hour with internal temperature about 70°C. After the addition the temperature of the mixture was raised and methanol was distilled off to a final temperature of 110°C (18 hours). The mixture was cooled to room temperature and the resulting lithium salt of 4-methoxycarbonylthiolan-3-one filtered and washed with toluene.
b) The product was suspended in water/dichloromethane and acidified with acetic acid (90g) to convert from lithium salt to free keto ester. The organic phase was dried and evaporated to yield the title compound (139g, 83%) as a pale yellow solid, m.p 40-41°C.

### EXAMPLE 2

### 4-Methoxycarbonylthiolan-3-one

Lithium metal (6.94g, 1M) was dissolved in methanol (400ml) then treated dropwise with 3-methoxycarbonylmethylthiopropanoic acid methyl ester (192g, 1M) over ½ hour. The mixture was then refluxed for 6 hours. The methanol was removed under reduced pressure and cold water (500 ml) added. The resulting lithium salt was removed by filtration. The crude lithium salt was slurried in water (1l) and treated with acetic acid (60 ml, 1m) and the keto ester extracted with di-isopropyl ether (2 x 500 ml). The organic phase was dried, evaporated and the residue recrystallised from hexane to give the title compound. (126g 79%) mp 40-1°C.

### EXAMPLE 3

### 2-Methoxycarbonylthiolan-3-one

3-Methoxycarbonylthiopropanoic acid methyl ester (19.2g, 0.1M) was added dropwise to a solution of lithium methoxide (5.7g, 0.15M) in methanol (150ml) at 25°C. After 2 hours tlc examination indicated that reaction was complete, giving a product more polar than that of Examples 1 and 2. (None of the product of Examples 1 and 2 was present). Acetic acid (9ml, 0.15M) in methanol (20ml) was added and the mixture poured onto water (500 ml). The product was extracted into di-isopropyl ether (2x250ml), and the organic phase washed with water and evaporated. Distillation gave the title compound (13.8g 86%) Bp. 90-5°C/lmbar.

### EXAMPLE 4

### 4-Methoxycarbonylthiolan-3-one

3-Methoxycarbonylthiopropanoic acid methyl ester (192g) was added to a solution of lithium methoxide (38g) in methanol (800 ml). The mixture was refluxed for 6 hours, cooled to 20°, treated with acetic acid (60 ml) and then poured on to water (21). The product was extracted with di-isopropyl ether and the organic extracts dried and evaporated. Recrystallisation of the rendue from hexane gave the title compound (130g, 81%) mp 40-1°C.

### EXAMPLE 5

### 4-Ethoxycarbonylthiolan-3-one

Following the procedure of Example 4 but using as reactants 220g 4-ethoxycarbonylthiopropanoic acid ethyl ester, 52g lithium ethoxide in 1l ethanol, the title compound was obtained, (yield 139g) Bp 120-2°/10mbar.

### EXAMPLE 6

### 2-Ethoxycarbonylthiolan-3-one

Following the procedure of Example 3 but using as reactants 22 g of 3-ethoxycarbonylmethylthiopropanoic acid ethyl ester and 7.8g lithium ethoxide in ethanol (250 ml) the title compound was obtained, 14.3g, Bp 96-8°/2mbar.

### EXAMPLE 7

### 4-Methoxycarbonylthiolan-3-one

A solution of 2-methoxycarbonylthiolan-3-one (16g, 0.1m) in methanol (50 ml) was added to lithium methoxide (3.8g, 0.1M) in methanol (50 ml) then the mixture was refluxed for 5 hours. The mixture was cooled and treated successively with acetic acid (6 ml) and water (250ml). The product was extracted with di-isopropyl ether (2x250ml) and the organic phase dried and evaporated. Recrystallisation of the residue from hexane gave the isomeric title compound (14.5g) Mp 40-1°C.

### EXAMPLE 8

### 4-n-Butoxycarbonylthiolan-3-one

Following the procedure of Example 3 but using as reactants 200g of 3-n-butoxycarbonylthiopropanoic acid n-butyl ester and 50g of lithium n-butoxide in 1 litre of n-butanol the title compound was obtained after 5 hours heating at 80°C.

## Claims (Claims for the following Contracting State(s): AT, BE, CH, DE, FR, GR, IT, LI, LU, NL, SE)

1. A process for the preparation of a compound of formula Ia or Ib or a mixture thereof 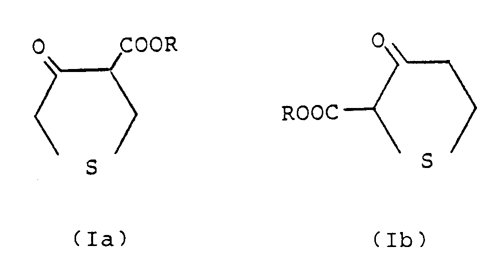 or lithium salts thereof which comprises cyclising a compound of formula 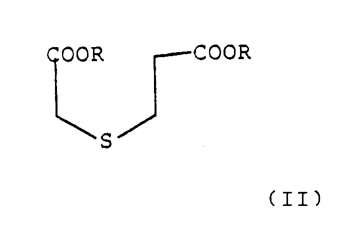 wherein COOR are the same or different ester functions, in the presence of a lithium alkoxide and if desired acidifying the lithium salt or salts obtained to give the compound of formula Ia or Ib or mixtures thereof.

2. A process according to claim 1 in which the cyclisation reaction is carried out for a time sufficient to produce the lithium salt of the compound of formula Ia substantially in excess of the lithium salt of the compound of formula Ib, and if desired acidifying to give the corresponding compound of formula Ia substantially in excess of the compound of formula Ib.

3. A process according to Claim 2 in which the cyclisation reaction is carried for a time sufficient to produce the lithium salt of the compound of formula Ia substantially free from the lithium salt of the compound of formula Ib.

4. A process according to Claim 2 or Claim 3 in which the cyclisation reaction is carried out at a temperature greater than about 50°C.

5. A process according to any one of Claims 1 to 4 in which isolation of the compound of formula Ia or the lithium salt thereof is effected about 3 hours or more after initiation of the process.

6. A process according to Claim 1 in which the cyclisation reaction is carried out for a time sufficiently short to produce the lithium salt of the compound of formula Ib substantially in excess of the lithium salt of the compound of formula Ia, and if desired acidifying to give the corresponding compound of formula Ib substantially in excess of the compound of formula Ia.

7. A process according to Claim 6 in which the cyclisation reaction is carried out for a time sufficiently short to produce the lithium salt of the compound of formula Ib substantially free from the lithium salt of the compound of formula Ia.

8. A process according to any one of Claims 1 to 7 in which the COOR groups represent the same or different alkyl or aralkyl ester functions.

9. A process according to Claim 8 in which the lithium alkoxide has the formula LiOR where R is an alkyl group the same as in the starting material of formula II.

10. A process according to any one of Claims 1 to 9 which is carried out in a solvent comprising an alcohol and optionally a hydrocarbon or a linear or cyclic ether.

11. The lithium salt of a compound of formula Ia or Ib 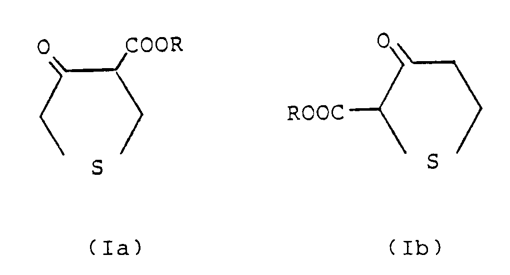 wherein COOR is as defined in Claim 1.

12. A process for preparing a compound of formula Ia or Ib as defined in Claim 1 which comprises acidifying a compound as claimed in Claim 11.

13. A compound as claimed in Claim 11 which is the lithium salt of 4-methoxycarbonyl- thiolan-3-one, or the lithium salt of 2-methoxycarbonyl- thiolan-2-one.

## Claims (Claims for the following Contracting State(s): ES)

1. A process for the preparation of a compound of formula Ia or Ib or a mixture thereof 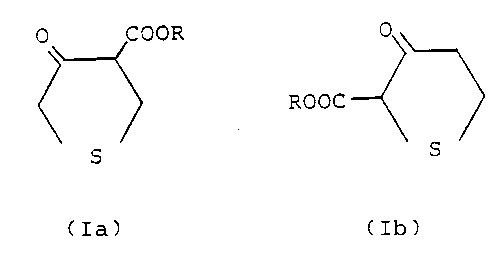 or lithium salts thereof which comprises cyclising a compound of formula 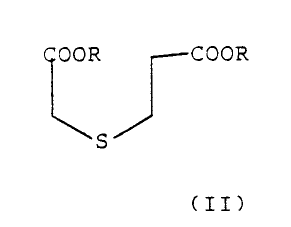 wherein COOR are the same or different ester functions, in the presence of a lithium alkoxide and if desired acidifying the lithium salt or salts obtained to give the compound of formula Ia or Ib or mixtures thereof.

2. A process according to claim 1 in which the cyclisation reaction is carried out for a time sufficient to produce the lithium salt of the compound of formula Ia substantially in excess of the lithium salt of the compound of formula Ib, and if desired acidifying to give the corresponding compound of formula Ia substantially in excess of the compound of formula Ib.

3. A process according to Claim 2 in which the cyclisation reaction is carried for a time sufficient to produce the lithium salt of the compound of formula Ia substantially free from the lithium salt of the compound of formula Ib.

4. A process according to Claim 2 or Claim 3 in which the cyclisation reaction is carried out at a temperature greater than about 50°C.

5. A process according to any one of Claims 1 to 4 in which isolation of the compound of formula Ia or the lithium salt thereof is effected about 3 hours or more after initiation of the process.

6. A process according to Claim 1 in which the cyclisation reaction is carried out for a time sufficiently short to produce the lithium salt of the compound of formula Ib substantially in excess of the lithium salt of the compound of formula Ia, and if desired acidifying to give the corresponding compound of formula Ib substantially in excess of the compound of formula Ia.

7. A process according to Claim 6 in which the cyclisation reaction is carried out for a time sufficiently short to produce the lithium salt of the compound of formula Ib substantially free from the lithium salt of the compound of formula Ia.

8. A process according to any one of Claims 1 to 7 in which the COOR groups represent the same or different alkyl or aralkyl ester functions.

9. A process according to Claim 8 in which the lithium alkoxide has the formula LiOR where R is an alkyl group the same as in the starting material of formula II.

10. A process according to any one of Claims 1 to 9 which is carried out in a solvent comprising an alcohol and optionally a hydrocarbon or a linear or cyclic ether.

## Patentansprüche (Patentansprüche für folgende(n) Vertragsstaat(en): AT, BE, CH, DE, FR, GR, IT, LI, LU, NL, SE)

1. Verfahren zum Herstellen einer Verbindung der Formel (Ia) oder (Ib) oder einer Mischung hievon 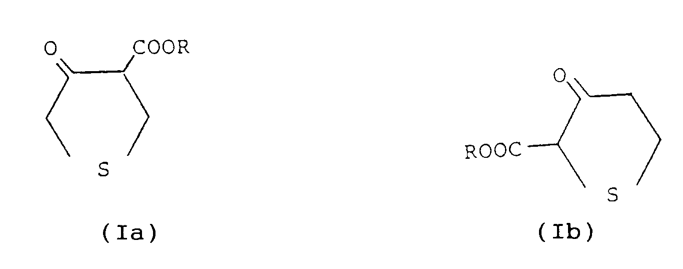 oder von Lithiumsalzen hievon, das das Cyclisieren einer Verbindung der Formel 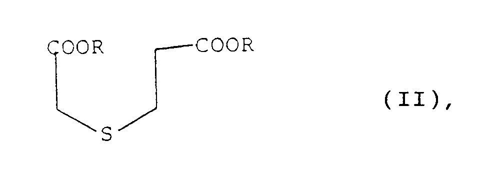 worin COOR die gleichen oder verschiedene Esterfunktionen darstellt, in Anwesenheit eines Lithiumalkoxids und, wenn gewünscht, das Ansäuern des oder der erhaltenen Lithiumsalze zum Erhalten der Verbindung der Formel (Ia) oder (Ib) oder von Mischungen hievon umfaßt.

2. Verfahren nach Anspruch 1, in dem die Cyclisierungsreaktion während eines Zeitraumes durchgeführt wird, der ausreichend ist, das Lithiumsalz der Verbindung der Formel (Ia) im wesentlichen im Überschuß zum Lithiumsalz der Verbindung der Formel (Ib) zu bilden, und, wenn gewünscht, angesäuert wird, wobei die entsprechende Verbindung der Formel (Ia) im wesentlichen im Überschuß zur Verbindung der Formel (Ib) erhalten wird.

3. Verfahren nach Anspruch 2, in dem die Cyclisierungsreaktion während eines Zeitraumes durchgeführt wird, der ausreichend ist, das Lithiumsalz der Verbindung der Formel (Ia) im wesentlichen frei vom Lithiumsalz der Verbindung der Formel (Ib) zu bilden.

4. Verfahren nach Anspruch 2 oder 3, in dem die Cyclisierungsreaktion bei einer Temperatur von höher als etwa 50°C durchgeführt wird.

5. Verfahren nach einem der Ansprüche 1 bis 4, in dem die Isolierung der Verbindung der Formel (Ia) oder des Lithiumsalzes hievon etwa 3 Stunden oder länger nach Einleitung des Verfahrens bewirkt wird.

6. Verfahren nach Anspruch 1, in dem die Cyclisierungsreaktion während eines Zeitraumes durchgeführt wird, der ausreichend kurz ist, das Lithiumsalz der Verbindung der Formel (Ib) im wesentlichen im Überschuß zum Lithiumsalz der Verbindung der Formel (Ia) zu bilden, und, wenn gewünscht, angesäuert wird, wobei die entsprechende Verbindung der Formel (Ib) im wesentlichen im Überschuß zur Verbindung der Formel (Ia) erhalten wird.

7. Verfahren nach Anspruch 6, in dem die Cyclisierungsreaktion während eines Zeitraumes durchgeführt wird, der ausreichend kurz ist, das Lithiumsalz der Verbindung der Formel (Ib) im wesentlichen frei vom Lithiumsalz der Verbindung der Formel (Ia) zu bilden.

8. Verfahren nach einem der Ansprüche 1 bis 7, in dem die Gruppen COOR die gleichen oder verschiedene Alkyl- oder Aralkylesterfunktionen darstellen.

9. Verfahren nach Anspruch 8, in dem das Lithiumalkoxid die Formel LiOR aufweist, worin R eine Alkylgruppe ist, die die gleiche ist wie im Ausgangsmaterial der Formel (II).

10. Verfahren nach einem der Ansprüche 1 bis 9, das in einem Lösungsmittel umfassend einen Alkohol und gegebenenfalls einen Kohlenwasserstoff oder einen linearen oder cyclischen Ether durchgeführt wird.

11. Lithiumsalz einer Verbindung der Formel (Ia) oder (Ib) 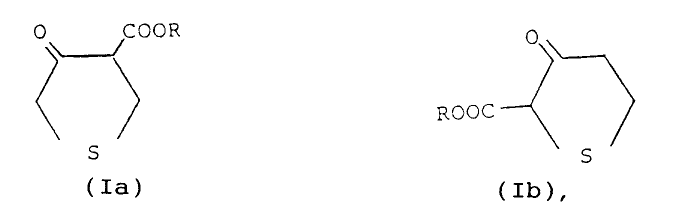 worin COOR wie in Anspruch 1 definiert ist.

12. Verfahren zum Herstellen einer Verbindung der Formel (Ia) oder (Ib), wie in Anspruch 1 definiert, das das Ansäuern einer Verbindung nach Anspruch 11 umfaßt.

13. Verbindung nach Anspruch 11, die das Lithiumsalz von 4-Methoxycarbonylthiolan-3-on oder das Lithiumsalz von 2-Methoxycarbonylthiolan-2-on ist.

## Patentansprüche (Patentansprüche für folgende(n) Vertragsstaat(en): ES)

1. Verfahren zum Herstellen einer Verbindung der Formel (Ia) oder (Ib) oder einer Mischung hievon 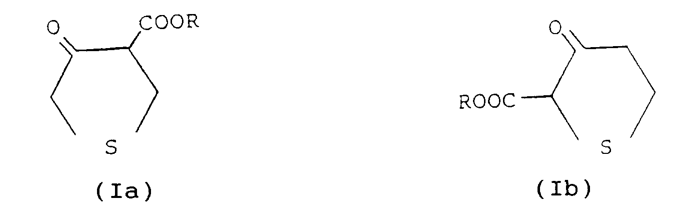 oder von Lithiumsalzen hievon, das das Cyclisieren einer Verbindung der Formel 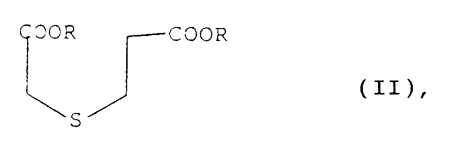 worin COOR die gleichen oder verschiedene Esterfunktionen darstellt, in Anwesenheit eines Lithiumalkoxids und, wenn gewünscht, das Ansäuern des oder der erhaltenen Lithiumsalze zum Erhalten der Verbindung der Formel (Ia) oder (Ib) oder von Mischungen hievon umfaßt.

2. Verfahren nach Anspruch 1, in dem die Cyclisierungsreaktion während eines Zeitraumes durchgeführt wird, der ausreichend ist, das Lithiumsalz der Verbindung der Formel (Ia) im wesentlichen im Überschuß zum Lithiumsalz der Verbindung der Formel (Ib) zu bilden, und, wenn gewünscht, angesäuert wird, wobei die entsprechende Verbindung der Formel (Ia) im wesentlichen im Überschuß zur Verbindung der Formel (Ib) erhalten wird.

3. Verfahren nach Anspruch 2, in dem die Cyclisierungsreaktion während eines Zeitraumes durchgeführt wird, der ausreichend ist, das Lithiumsalz der Verbindung der Formel (Ia) im wesentlichen frei vom Lithiumsalz der Verbindung der Formel (Ib) zu bilden.

4. Verfahren nach Anspruch 2 oder 3, in dem die Cyclisierungsreaktion bei einer Temperatur von höher als etwa 50°C durchgeführt wird.

5. Verfahren nach einem der Ansprüche 1 bis 4, in dem die Isolierung der Verbindung der Formel (Ia) oder des Lithiumsalzes hievon etwa 3 Stunden oder länger nach Einleitung des Verfahrens bewirkt wird.

6. Verfahren nach Anspruch 1, in dem die Cyclisierungsreaktion während eines Zeitraumes durchgeführt wird, der ausreichend kurz ist, das Lithiumsalz der Verbindung der Formel (Ib) im wesentlichen im Überschuß zum Lithiumsalz der Verbindung der Formel (Ia) zu bilden, und, wenn gewünscht, angesäuert wird, wobei die entsprechende Verbindung der Formel (Ib) im wesentlichen im Überschuß zur Verbindung der Formel (Ia) erhalten wird.

7. Verfahren nach Anspruch 6, in dem die Cyclisierungsreaktion während eines Zeitraumes durchgeführt wird, der ausreichend kurz ist, das Lithiumsalz der Verbindung der Formel (Ib) im wesentlichen frei vom Lithiumsalz der Verbindung der Formel (Ia) zu bilden.

8. Verfahren nach einem der Ansprüche 1 bis 7, in dem die Gruppen COOR die gleichen oder verschiedene Alkyl- oder Aralkylesterfunktionen darstellen.

9. Verfahren nach Anspruch 8, in dem das Lithiumalkoxid die Formel LiOR aufweist, worin R eine Alkylgruppe ist, die die gleiche ist wie im Ausgangsmaterial der Formel (II).

10. Verfahren nach einem der Ansprüche 1 bis 9, das in einem Lösungsmittel umfassend einen Alkohol und gegebenenfalls einen Kohlenwasserstoff oder einen linearen oder cyclischen Ether durchgeführt wird.

## Revendications (Revendications pour l'(les) Etat(s) contractant(s) suivant(s): AT, BE, CH, DE, FR, GR, IT, LI, LU, NL, SE)

1. Procédé de préparation d'un composé de formule Ia ou Ib ou d'un mélange de ceux-ci : 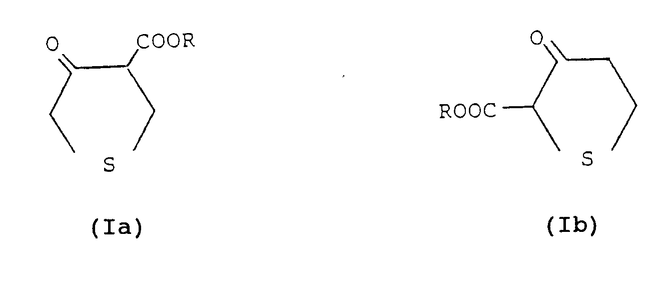 ou des sels de lithium de ceux-ci, qui comprend la cyclisation d'un composé de formule : 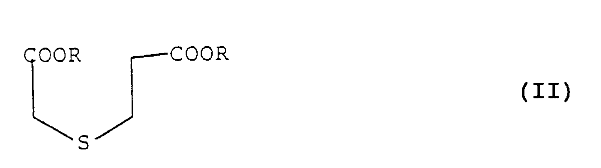 où COOR représente des fonctions ester identiques ou différentes, en présence d'un alcoolate de lithium et, si la chose est souhaitée, l'acidification du ou des sels de lithium obtenus pour former le composé de formule Ia ou Ib ou leurs mélanges.

2. Procédé suivant la revendication 1, dans lequel la réaction de cyclisation est exécutée pendant un temps suffisant pour produire le sel de lithium du composé de formule Ia en excès sensible sur le sel de lithium du composé de formule Ib et, si la chose est souhaitée, l'acidification est exécutée pour former le composé correspondant de formule Ia en excès sensible sur le composé de formule Ib.

3. Procédé suivant la revendication 2, dans lequel la réaction de cyclisation est exécutée pendant un temps suffisant pour produire le sel de lithium du composé de formule Ia sensiblement exempt du sel de lithium du composé de formule Ib.

4. Procédé suivant la revendication 2 ou 3, dans lequel la réaction de cyclisation est exécutée à une température supérieure à environ 50°C.

5. Procédé suivant l'une quelconque des revendications 1 à 4, dans lequel l'isolement du composé de formule Ia ou de son sel de lithium est exécuté environ 3 heures ou davantage après l'initiation du procédé.

6. Procédé suivant la revendication 1, dans lequel la réaction de cyclisation est exécutée pendant un temps suffisamment court pour produire le sel de lithium du composé de formule Ib en excès sensible sur le sel de lithium du composé de formule Ia et, si la chose est souhaitée, l'acidification est exécutée pour former le composé correspondant de formule Ib en excès sensible sur le composé de formule Ia.

7. Procédé suivant la revendication 6, dans lequel la réaction de cyclisation est exécutée pendant un temps suffisamment court pour produire le sel de lithium du composé de formule Ib sensiblement exempt du sel de lithium du composé de formule Ia.

8. Procédé suivant l'une quelconque des revendications 1 à 7, dans lequel les radicaux COOR représentent des fonctions ester alcoylique ou aralcoylique identiques ou différentes.

9. Procédé suivant la revendication 8, dans lequel l'alcoolate de lithium est de formule LiOR où R est un radical alcoyle qui est le même que dans le composé de départ de formule II.

10. Procédé suivant l'une quelconque des revendications 1 à 9, qui est exécuté dans un solvant comprenant un alcool et facultativement un hydrocarbure ou un éther linéaire ou cyclique.

11. Sel de lithium d'un composé de formule Ia ou Ib : 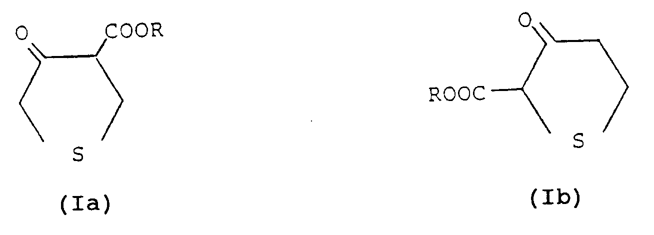 où COOR est tel que défini dans la revendication 1.

12. Procédé de préparation d'un composé de formule Ia ou Ib tel que défini dans la revendication 1, qui comprend l'acidification d'un composé suivant la revendication 11.

13. Composé suivant la revendication 11, qui est le sel de lithium de la 4-méthoxycarbonylthiolan-3-one ou le sel de lithium de la 2-méthoxycarbonylthiolan-2-one.

## Revendications (Revendications pour l'(les) Etat(s) contractant(s) suivant(s): ES)

1. Procédé de préparation d'un composé de formule Ia ou Ib ou d'un mélange de ceux-ci : 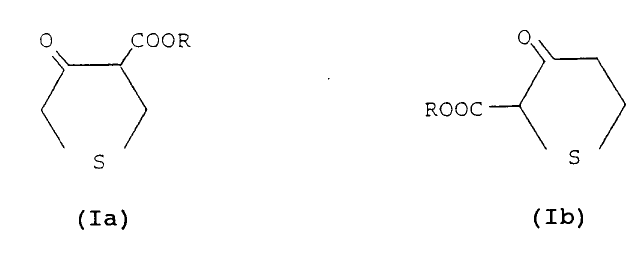 ou des sels de lithium de ceux-ci, qui comprend la cyclisation d'un composé de formule : 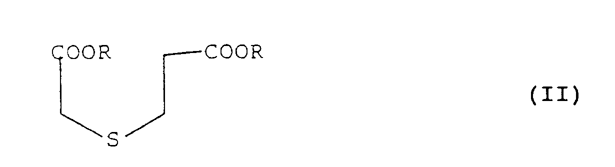 où COOR représente des fonctions ester identiques ou différentes, en présence d'un alcoolate de lithium et, si la, chose est souhaitée, l'acidification du ou des sels de lithium obtenus pour former le composé de formule Ia ou Ib ou leurs mélanges.

2. Procédé suivant la revendication 1, dans lequel la réaction de cyclisation est exécutée pendant un temps suffisant pour produire le sel de lithium du composé de formule Ia en excès sensible sur le sel de lithium du composé de formule Ib et, si la chose est souhaitée, l'acidification est exécutée pour former le composé correspondant de formule Ia en excès sensible sur le composé de formule Ib.

3. Procédé suivant la revendication 2, dans lequel la réaction de cyclisation est exécutée pendant un temps suffisant pour produire le sel de lithium du composé de formule Ia sensiblement exempt du sel de lithium du composé de formule Ib.

4. Procédé suivant la revendication 2 ou 3, dans lequel la réaction de cyclisation est exécutée à une température supérieure à environ 50°C.

5. Procédé suivant l'une quelconque des revendications 1 à 4, dans lequel l'isolement du composé de formule Ia ou de son sel de lithium est exécuté environ 3 heures ou davantage après l'initiation du procédé.

6. Procédé suivant la revendication 1, dans lequel la réaction de cyclisation est exécutée pendant un temps suffisamment court pour produire le sel de lithium du composé de formule Ib en excès sensible sur le sel de lithium du composé de formule Ia et, si la chose est souhaitée, l'acidification est exécutée pour former le composé correspondant de formule Ib en excès sensible sur le composé de formule Ia.

7. Procédé suivant la revendication 6, dans lequel la réaction de cyclisation est exécutée pendant un temps suffisamment court pour produire le sel de lithium du composé de formule Ib sensiblement exempt du sel de lithium du composé de formule Ia.

8. Procédé suivant l'une quelconque des revendications 1 à 7, dans lequel les radicaux COOR représentent des fonctions ester alcoylique ou aralcoylique identiques ou différentes.

9. Procédé suivant la revendication 8, dans lequel l'alcoolate de lithium est de formule LiOR où R est un radical alcoyle qui est le même que dans le composé de départ de formule II.

10. Procédé suivant l'une quelconque des revendications 1 à 9, qui est exécuté dans un solvant comprenant un alcool et facultativement un hydrocarbure ou un éther linéaire ou cyclique.
